# EUROPÄISCHE PATENTSCHRIFT

(11) **EP 1 890 994 B1**
(45) Veröffentlichungstag und Bekanntmachung des Hinweises auf die Patenterteilung: **05.10.2011**
(21) Anmeldenummer: 06743005.8
(22) Anmeldetag: 20.05.2006
(51) Int. Cl.: A61K 31/616, C07C 69/86, A61K 31/198, A61P 11/06, A61P 9/00

(54) **STABILER WIRKSTOFFKOMPLEX VON SALZEN DER O-ACETYLSALICYLSÄURE MIT BASISCHEN AMINOSÄUREN UND GLYCIN**
STABILE ACTIVE INGREDIENT COMPLEX OF SALTS OF THE O-ACETYLSALICYLIC ACID WITH BASIC AMINO ACIDS AND GLYCINE
COMPLEXE ACTIF STABLE DE SELS DE L'ACIDE O-ACETYLSALICYLIQUE CONTENANT DES ACIDES AMINES BASIQUES ET DE LA GLYCINE

(30) Priorität: 02.06.2005 DE 102005025283
(43) Veröffentlichungstag der Anmeldung: 27.02.2008
(73) Patentinhaber: Bayer Pharma Aktiengesellschaft, 13353 Berlin (DE)
(72) Erfinder: FRANCKOWIAK, Gerhard, 42115 Wuppertal (DE); LEDWOCH, Wolfram, 40764 Langenfeld (DE); SCHWEINHEIM, Eberhard, 51375 Leverkusen (DE); HAYAUCHI, Yutaka, 51373 Leverkusen (DE)
(86) Internationale Anmeldenummer: PCT/EP2006/004799
(87) Internationale Veröffentlichungsnummer: WO 2006/128600

(56) Entgegenhaltungen:
- EP-A2- 0 089 198
- WO-A-2005/115404
- WO-A2-02/05782
- WO-A2-03/059323
- GB-A- 2 051 573
- DATABASE WPI Week 198129 Derwent Publications Ltd., London, GB; AN 1981-52701D XP002415114 & JP 56 065816 A (GREEN CROSS CORP) 3. Juni 1981 (1981-06-03)
- NAKAI T ET AL: "Plasma cortisol levels in umbilical cord plasma and its change during the first two hours following delivery (Japanese)" FOLIA ENDOCRINOL.JAP. 1976, Bd. 52, Nr. 5, 1976, Seiten 595-602, XP008073655
- DATABASE WPI Week 200350 Derwent Publications Ltd., London, GB; AN 2003-530823 XP002417812 & RU 2 203 064 C2 (KAMENEV V F) 27. April 2003 (2003-04-27)

## Beschreibung

Die vorliegende Erfindung betrifft stabile Wirkstoffkomplexe von Salzen der o-Acetylsalicylsäure mit basischen Aminosäuren und Glycin, ein Verfahren zu deren Herstellung sowie deren Verwendung als Arzneimittel.

Die analgetische Wirkung von o-Acetylsalicylsäure (Aspirin®) wird seit langem therapeutisch genutzt. So wird o-Acetylsalicylsäure als Analgetikum, Antipyretikum, Antirheumatikum, sowie als nichtsteroidaler entzündungshemmender Wirkstoff beispielsweise zur Behandlung von Arthritis, Neuralgien und Myalgien eingesetzt.

Allerdings ist o-Acetylsalicylsäure nur begrenzt löslich und somit die Resorptionsgeschwindigkeit limitiert. Gerade bei Schmerzen, vor allem bei Migräne, ist für den therapeutischen Effekt ein rasches Anfluten des Wirkstoffs im Körper erwünscht und erforderlich. Bisher konnte dies nur durch geeignete Darreichungsformen, wie z.B. gepufferten Brausetabletten oder Kautabletten erreicht werden.

Eine Möglichkeit, schnell hohe Blutspiegel des Wirkstoffs zu erreichen, ist die Vergrößerung der Lösegeschwindigkeit des Wirkstoffs selbst. Dies ist mit Salzen -der o-Acetylsalicylsäure zu erreichen. Zudem ist bei längerer oraler Verabreichung eine gute Verträglichkeit der o-Acetylsalicylate hervorzuheben.

Bekannte Salze der Acetylsalicylsäure sind unter anderem Salze der Acetylsalicylsäure mit basischen Aminosäuren. Als basische Aminosäuren werden insbesondere L-Lysin, D,L-Lysin oder Arginin eingesetzt. Es kann auch ein gewisser Anteil an Glycin zugesetzt werden. Therapeutisch wird das Salz der Acetylsalicylsäure (ASS) mit der Aminosäure Lysin eingesetzt. Bei dem gängigsten Medikament mit ASS-Lysinat handelt es sich um eine Darreichungsform zur parenteralen Applikation enthaltend zusätzlich Glycin. Es ist unter dem Namen Aspisol® (bis Mitte 2005) im Handel. Das Glycin wird dem ASS-Lysinat fest zugegeben, so dass ein Gemenge aus ASS-Lysinat und Glycin vorliegt.

Ein gewisser Nachteil der o-Acetylsalicylate lag bisher in deren unzureichenden Stabilität. Einerseits resultiert daraus eine eingeschränkte Haltbarkeit der aus diesen Salzen hergestellten pharmazeutischen Präparate. Andererseits kann eine gegebenenfalls erforderliche Sterilisierung des Wirkstoffs wegen der unzureichenden thermischen Stabilität dieser Salze nicht über eine Hitzesterilisierung durchgeführt werden, sondern muß auf anderen Wegen wie beispielsweise durch Begasung mit Ethylenoxid erfolgen.

Die geringe Stabilität der o-Acetylsalicylate ist auf eine dem Fachmann bekannte Rückreaktion des Produkts zu o-Acetylsalicylsäure und der entsprechenden Aminosäure zurückzuführen. Die Aminosäure reagiert dann mit der o-Acetylsalicylsäure unter Abspaltung der Acetylgruppe (Amidolyse) und Freisetzung von Salicylsäure. Die Anwesenheit von freier Salicylsäure in pharmazeutischen Präparaten ist jedoch unerwünscht und deshalb auf einen geringen, akzeptablen Wert zu begrenzen (Arch. Pharm. 318, 120, 1985).

In der WO 02/005782 und der WO 03/059323 werden Salze von o-Acetylsalicylsäure mit basischen Aminosäuren beschrieben, die eine erhöhte Stabilität aufweisen und deshalb die Nachteile der bisher bekannten o-Acetylsalicylate hinsichtlich Lagerung und/oder Sterilisierbarkeit nicht aufweisen. Die Salze werden nach einem besonderen Verfahren hergestellt und zeichnen sich bei einer mit einem Malvern 2600D-Gerät unter Standardbedingungen gemessenen Korngrößenverteilung durch eine mittlere Korngröße oberhalb einer Korngröße von 160 µm und einen Anteil von mehr als 60 % der Teilchen mit einer Korngröße in einem Bereich von 100 bis 200 µm aus. Sie können einen gewissen Gehalt an zugegebenem Glycin aufweisen. Des weiteren wird in der WO 02/005782 und der WO 03/059323 beschrieben, daß der Zusatz des Glycins nicht erforderlich ist und die Art der Zugabe keinen Einfluss auf die Eigenschaften des o-Acetylsalicylats hat, insbesondere hat die Anwesenheit von Glycin keinen Einfluss auf die Stabilität der o-Acetylsalicylate.

Überraschenderweise wurde nun gefunden, daß die Art der Zugabe des Glycins bei der Herstellung des o-Acetylsalicylats einen erheblichen Einfluß auf die Eigenschaften des o-Acetylsalicylats hat.

Die vorliegende Erfindung betrifft Wirkstoffkomplexe von Salzen der o-Acetylsalicylsäure mit basischen Aminosäuren und Glycin, gemäss der Ansprüchen.

Der erfindungsgemäße Wirkstoffkomplex zeichnet sich durch eine hohe Stabilität aus und weist eine charakteristische Kristallform auf. Dieses wird durch die beiliegenden Abbildungen näher erläutert:
- Fig. 1:: Elektronenmikroskopische Aufnahme der Kristalle von Aspisol (Handelsware bis Mitte 2005).
- Fig. 2:: Elektronenmikroskopische Aufnahme eines Kristalls des erfindungsgemäßen Wirk- stoffkomplexes des D,L-Lysinats der o-Acetylsalicylsäure mit Glycin gemäß Beispiel 1.
- Fig. 3:: Stabilitätsdaten von Aspisol (Handelsware bis Mitte 2005) und Beispiel 1, Lagerbe- dingungen: 25°C/60 % relative Luftfeuchte - Gemessen wird die Bildung von Salicyl- säure.

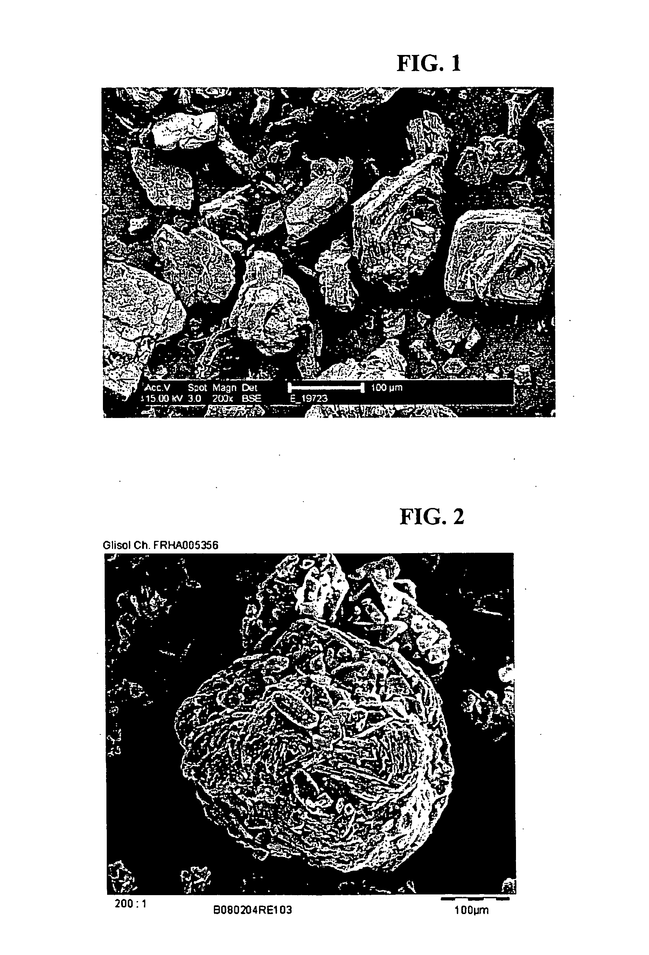

Fig. 1 zeigt Kristalle von Aspisol (Handelsware bis Mitte 2005), in dem das D,L-Lysinat der o-Acetylsalicylsäure und Glycin als Gemenge nebeneinander vorliegen. Dieses ist in dem Herstellungsverfahren begründet, in dem das Glycin und das D,L-Lysinat der o-Acetylsalicylsäure als Festsubstanzen zum Schluß miteinander trocken vermengt werden. Fig. 2 spiegelt dagegen die deutlich unterschiedliche Kristallform des erfindungsgemäßen Beispiels 1 wider.

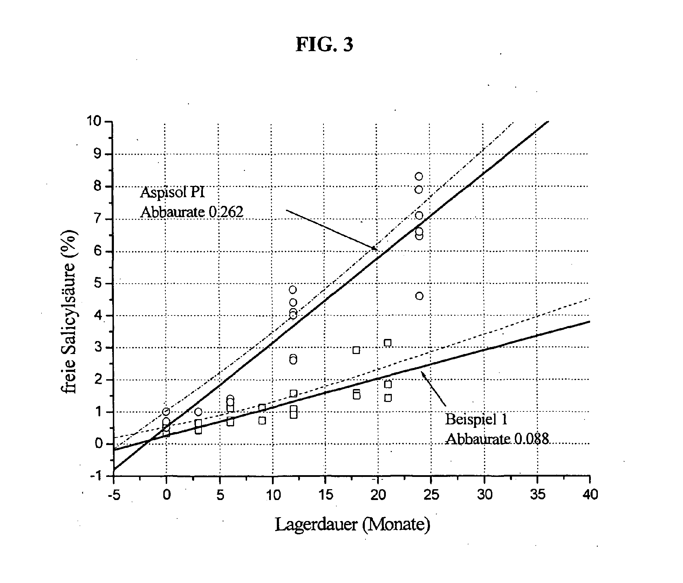

Fig. 3 zeigt die deutlich erhöhte Stabilität von Beispiel 1 gegenüber Aspisol (Handelsware bis Mitte 2005). Die freigesetzte Salicylsäure ist beispielsweise nach 30 Monaten um ein Drittel geringer.

Die erfindungsgemäß geeigneten basischen Aminosäuren zur Bildung der o-Acetylsalicylate können in der L- oder in der D-Konfiguration auftreten oder auch als Gemisch von D- und L-Form. Der Begriff "Aminosäuren" bezeichnet erfindungsgemäß insbesondere die in der Natur vorkommenden L-Aminosäuren, umfasst darüber hinaus aber auch deren Solvate wie beispielsweise Hydrate, Homologe, Isomere und Derivate. Als Beispiel für Isomere können Enantiomere genannt werden. Derivate können beispielsweise mit Schutzgruppen versehene Aminosäuren sein. Als typische Beispiele für basische Aminosäuren seien genannt: Lysin, Arginin, Ornithin, Diaminobuttersäure. Das Salz der o-Acetylsalicylsäure mit Lysin ist bevorzugt genannt.

Durch den erfindungsgemäßen Begriff "Wirkstoffkomplex" wird ein Produkt beschrieben, das aus in enger Verbindung miteinander verwachsenen Kristallen von einem Salz der o-Acetylsalicylsäure mit einer basischen Aminosäure und Glycin besteht. Dabei liegt kein Kristallgemisch der einzelnen Komponenten o-Acetylsalicylat und Glycin vor.

Der Gehalt an Glycin in den erfindungsgemäßen Kristallen beträgt 8 bis 12, bevorzugt 9 bis 11, besonders bevorzugt 10 Gewichtsprozente bezogen auf den Wirkstoffkomplex.

Die vorteilhaften Eigenschaften der vorliegenden Erfindung werden unabhängig von der Korngröße des erfindungsgemäßen Wirkstoffkomplexes beobachtet. Die Korngrößenverteilung des erfindungsgemäßen Wirkstoffkomplexes weist eine mittlere Korngröße von unterhalb 100 µm, besonders bevorzugt von unterhalb 70 µm auf.

### Herstellung:

Gemäß der vorliegenden Erfindung werden Lösungen der Reaktionspartner, d.h. von o-Acetylsalicylsäure und der entsprechenden Aminosäure, bei einer Temperatur kleiner gleich 40°C, vorzugsweise von 20 bis 35°C, unter Normaldruck möglichst rasch, bevorzugt in weniger als 20 Minuten, zusammengegeben und zu einer homogenen Phase vermischt, so daß die Temperatur 40°C nicht übersteigt. Zu der so hergestellten homogenen Mischung werden gegebenenfalls Impfkristalle zugesetzt, auf -5 bis 10°C, bevorzugt auf 0 bis 5°C abgekühlt und bei dieser Temperatur 2 bis 8 Stunden, bevorzugt 3 bis 5 Stunden gerührt. Es wird mit gekühltem Aceton und mit der benötigten Menge an gegebenenfalls gekühltem Glycin versetzt. Die Suspension sollte zur Vervollständigung der Kristallisation mindestens eine Stunde unter den vorstehend angegebenen Bedingungen gehalten werden. Erfindungsgemäß bevorzugt ist eine Kristallisationsdauer von 1 bis 10 Stunden unter den vorstehend angegebenen Bedingungen, wobei ein Zeitraum von 1 bis 8 Stunden besonders bevorzugt ist. Es ist gemäß der vorliegenden Erfindung sehr wichtig, daß die Temperatur während des Kristallisationsvorgangs in möglichst engen Grenzen gehalten wird. Die Temperatur darf 5°C nicht übersteigen und sollte vorzugsweise unterhalb von 3°C, besonders bevorzugt zwischen 0 und 2°C, gehalten werden. Als Impfkristalle können Kristalle des gewünschten Produkts verwendet werden. Die Kristallisation erfolgt bevorzugt unter Normaldruck.

Das Kristallisat wird anschließend auf herkömmliche Weise isoliert, beispielsweise durch Filtrieren oder Zentrifugieren. Der Feststoff wird mehrfach mit organischen Lösungsmitteln gewaschen, wobei erfindungsgemäß Alkohole wie beispielsweise Ethanol und/oder Ketone wie Aceton oder Mischungen von Alkoholen und/oder Ketonen, beispielsweise Mischungen von Ethanol und Aceton, oder die Verwendung verschiedener derartiger Lösungsmittel bevorzugt ist.

Der Feststoff wird anschließend unter vermindertem Druck getrocknet. Hierbei sollte die Temperatur unter 50°C, vorzugsweise unter 40°C und besonders bevorzugt unter 35°C gehalten werden. Es sollte ein Druck von weniger als 100 mbar, vorzugsweise von weniger als 50 mbar an den Feststoff angelegt werden. Die Trocknung kann unter herkömmlichen Bedingungen erfolgen, beispielsweise in einem Trockengerät.

Als Lösungsmittel für die Reaktionspartner kommen Wasser beziehungsweise mit Wasser mischbare organische Lösungsmittel wie beispielsweise Alkohole wie Methanol, Ethanol oder isoPropanol, insbesondere Ethanol, Ether wie Tetrahydrofuran (THF) oder Ketone wie Aceton, oder Gemische der genannten Lösungsmittel in Frage. Bevorzugt sind Wasser, Ethanol oder ein Gemisch aus beiden.

Bevorzugt wird die o-Acetylsalicylsäure in Ethanol gelöst und die Aminosäure, bevorzugt Lysin, besonders bevorzugt D,L-Lysin Monohydrat in Wasser gelöst zusammengegeben.

Die Reaktionspartner werden in solchen Mengen eingesetzt, daß die basische Aminosäure in leichtem Überschuss bezogen auf die Mol o-Acetylsalicylsäure vorliegt. Erfindungsgemäß bevorzugt ist ein Molverhältnis von o-Acetylsalicylsäure zu Aminosäure von 1:1,05 bis 1:1,5, wobei ein Verhältnis von o-Acetylsalicylsäure zu Aminosäure von 1:1,05 bis 1:1,2 besonders bevorzugt ist.

Gemäß der vorliegenden Erfindung sollte die o-Acetylsalicylsäurelösung einen Gehalt von 1 bis 10 Gew.-%, vorzugsweise 5 bis 10 Gew.-% und insbesondere bevorzugt von 6 bis 8 Gew.-% o-Acetylsalicylsäure aufweisen. Die Lösung der basischen Aminosäure sollte einen Gehalt von 10 bis 40 Gew.-%, vorzugsweise 15 bis 35 Gew.-% und insbesondere bevorzugt von 20 bis 30 Gew.-% Aminosäure aufweisen.

Das Glycin kann gemäß der vorliegenden Erfindung als Lösung in Wasser oder einem mit Wasser mischbaren organischen Lösungsmittel, wobei als organische Lösungsmittel die vorstehend beschriebenen Lösungsmittel verwendet werden können, der Reaktionsmischung aus den Reaktionspartnern zugegeben werden.

Das Glycin kann gemäß der vorliegenden Erfindung aber auch in Form einer Suspension zugegeben werden. Die Glycin-Suspension kann auf herkömmliche Weise hergestellt werden. Erfindungsgemäß bevorzugt ist die Herstellung einer Glycin-Suspension aus einem Lösungsmittelgemisch aus Wasser und einem Alkohol wie beispielsweise Ethanol.

Ebenso wichtig ist beim erfindungsgemäßen Verfahren das Einhalten einer bestimmten Rührenergie während der Kristallisation. Die homogene Mischung der Ausgangsprodukte darf nur sanft gerührt werden. Die anzulegende Rührenergie sollte nicht größer als 0,1 W pro Liter Reaktionsmedium sein. Erfindungsgemäß bevorzugt ist eine angelegte Rührenergie von 0,04 bis 0,06 W pro Liter Reaktionsmedium. Als Rührer kommen alle herkömmlichen entsprechend regulierbaren Rührgeräte wie beispielsweise ein Rührwerkbehälter mit Stromstörer in Betracht.

Das erfindungsgemäße Verfahren kann auch vollständig unter sterilen Bedingungen durchgeführt werden. Die hierfür erforderlichen Abweichungen von der obigen Vorgehensweise beispielsweise hinsichtlich Sterilisierung der Ausgangsverbindungen sowie der eingesetzten Apparaturen sind dem Fachmann bekannt.

### Arzneimittel:

Zur vorliegenden Erfindung gehören auch pharmazeutische Zubereitungen, die neben nichttoxischen, inerten pharmazeutisch geeigneten Hilfsstoffen den erfindungsgemäßen Wirkstoffkomplex enthalten, sowie Verfahren zur Herstellung dieser Zubereitungen.

Der Wirkstoffkomplex kann systemisch und/oder lokal wirken. Zu diesem Zweck kann er auf geeignete Weise appliziert werden, wie beispielsweise oral oder parenteral. Für diese Applikationswege kann der Wirkstoffkomplex in geeigneten Applikationsformen verabreicht werden.

Für die orale Applikation eignen sich bekannte, den Wirkstoffkomplex schnell und/oder modifiziert abgebende Applikationsformen, wie beispielsweise Tabletten (nichtüberzogene sowie überzogene Tabletten, beispielsweise magensaftresistente Überzüge, FDT (Fast dissolve Tabletten), Brausetabletten, Kautabletten), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan oder intraperitoneal). Für die parenterale Applikation eignen sich als Applikationsformen u.a. Injektions- und Infusionszubereitungen in Form von Lösungen, Suspensionen oder Emulsionen.

Bevorzugt ist eine Applikation als Injektions- und Infusionszubereitung. Dabei kann es sich um bereits fertige Injektions- oder Infusionszubereitungen in Form von Lösungen, Suspensionen oder Emulsionen handeln oder aber auch um Applikationsformen, in denen der Wirkstoffkomplex beispielsweise als Lyophilisat oder steriles Pulver getrennt von dem Injektions- oder Infusionslösungsmittel vorliegt und erst kurz vor der Applikation durch Mischen mit dem Lösungsmittel beispielsweise Wasser die fertige Injektions- oder Infusionszubereitung hergestellt werden kann.

Die topische Applikation in Form von Suppositorien oder von transdermalen Systemen (z.B. Pflaster, ETS-Systeme) sowie in Cremes, Salben, Gels, Sprays oder gelöst in organischen oder anorganischen Lösungsmitteln sind weitere Anwendungsmöglichkeiten,

Der erfindungsgemäße Wirkstoffkomplex kann in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und / oder Geruchskorrigentien.

Im allgemeinen hat es sich sowohl in der Human- als auch in der Veterinärmedizin als vorteilhaft erwiesen, den erfindungsgemäßen Wirkstoffkomplex in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den erfindungsgemäßen Wirkstoffkomplex vorzugsweise in Mengen von etwa 1 bis etwa 80, insbesondere 3 bis 30 mg/kg Körpergewicht.

### Verwendung:

Die erfindungsgemäßen Arzneimittel können als Analgetikum, Antipyretikum, Antirheumatikum, sowie als nichtsteroidale entzündungshemmende Arzneimittel beispielsweise zur Behandlung von Erkrankungen des rheumatischen Formenkreises, Arthritiden, Neuralgien, Myalgien und/oder Migräne eingesetzt werden. Insbesondere können sie aber auch als Thrombozytenaggregationshemmer in der Prävention und Therapie kardio- und cerebrovaskulärer Erkrankungen eingesetzt werden, z.B. bei ischämischen Herzkrankheiten, Schlaganfall (Stroke), stabiler und instabiler Angina pectoris, myocardialem Infarkt (z.B. akutem Myokardinfarkt), Bypass-Operationen, PTCA (Perkutane Transluminale Coronar-Angioplastie) und/oder Stent-Implantation. Weitere Anwendungsgebiete sind die Stimulierung des Immunsystems bei HIV-Patienten und die Tumorprophylaxe (z.B. Kolon-, Oesophagus- oder Lungenkarzinom), Verlangsamung des kognitiven Verfalls bei dementiellem Syndrom (z.B. Alzheimer-Erkrankung), Hemmung der Gallensteinbildung sowie die Behandlung diabetischer Erkrankungen.

Des weiteren zeigt der erfindungsgemäße Wirkstoffkomplex anti-asthmatische Aktivität, wenn er inhaliert wird.

### Ausführungsbeispiele:

### Beispiel 1: D,L-Lysinacetylsalicylat mit 10 % Glycin

In einen sterilen und pyrogenfreien Rührwerkbehälter mit Stromstörer gibt man über einen Sterilfilter eine pyrogenfreie Lösung von 40,0 kg o-Acetylsalicylsäure in 500 kg Ethanol. Bei 20 bis 30°C fügt man unter Rühren und Kühlen innerhalb kurzer Zeit (unter 15 Minuten) eine sterilfiltrierte und pyrogenfreie Lösung von 36,4 kg D,L-Lysin Monohydrat in 110 kg pyrogenfreiem Wasser hinzu, so dass eine Temperatur von 35°C nicht überschritten wird. Es werden mindestens 20 g an sterilen Impfkristallen hinzugefügt und die bereits kristallisierende Mischung unter reduzierter Rührerdrehzahl auf 2°C gekühlt. Danach werden 490 kg pyrogenfreies und temperiertes Aceton und eine aseptisch und temperierte vorbereitete Suspension von 8,0 kg Glycin in 25,0 kg pyrogenfreiem Wasser und 90 kg Ethanol hinzugefügt. Unter weiterem Kühlen bei 2°C wird die Suspension 1 bis 8 Stunden weiter gerührt. Erst dann wird das Kristallgemisch unter aseptischen Bedingungen auf einem Filter oder einer Zentrifuge isoliert. Das Feuchtprodukt wird auf dem Trennapparat mit pyrogenfreiem Ethanol und Aceton gewaschen und unter aseptischen Bedingungen bis zu einem Druck ≤ 50 mbar und einer Temperatur von nicht mehr als 40°C getrocknet. Anschließend wird das Fertigprodukt in Gebinde mit PE-Inlinern abgefüllt und versiegelt. Es werden 60 bis 70 kg (75 bis 87 % d. Theorie) des Titelprodukts mit einer Restfeuchte < 0,3 % mit einer mittleren Korngröße von 41 µm erhalten.

### Schmelzpunktbestimmungen durch DSC (Differential Scanning Calorimetry):

Die Schmelzpunktbestimmungen durch DSC werden mit dem Gerät Pyris-1 der Firma PerkinElmer mit einer Heizrate von 20 K/min. durchgeführt. Trockener Stickstoff wird als Schutzgas verwendet. Die charakteristischen DSC-Kurven von Aspisol® (Handelsware bis Mitte 2005) und Produkt gemäß Beispiel 1 zeigen zwei Peaks, einen endothermischen gefolgt von einem exothermischen. Der endothermische Peak resultiert aus dem Schmelzprozeß, wogegen der exothermische Peak aus einer Überlappung von Zerfall und partieller Kristallisation eines Zerfallprodukts (z.B. Acetylsalicylsäure) in der geschmolzenen Phase resultiert.

**Tabelle 1:**

| Charge | Peaktemperatur [°C] (endothermisch) | Peaktemperatur [°C] (exothermisch) |
|---|---|---|
| Aspisol® (Handelsware bis Mitte 2005) | 144,4 ± 2,48 | 149,0 ± 2,0 |
| Beispiel 1 | 147,9 ± 1,44 | 153,0 ± 1,0 |

## Patentansprüche

1. Wirkstoffkomplex bestehend aus einem Salz der o-Acetylsalicylsäure mit einer basischen Aminosäure und Glycin, **dadurch gekennzeichnet, dass** die Korngrößenverteilung eine mittlere Korngröße unterhalb von 100 µm aufweist.

2. Wirkstoffkomplex nach Anspruch 1, **dadurch gekennzeichnet, dass** der Wirkstoffkomplex zu 8 bis 12 Gewichtsprozenten aus Glycin besteht.

3. Wirkstoffkomplex nach einem der Ansprüche 1 bis 2 mit Lysin als basische Aminosäure.

4. Wirkstoffkomplex nach einem der Ansprüche 1 bis 3 mit D,L-Lysin als basische Aminosäure.

5. Wirkstoffkomplex nach Anspruch 4, **dadurch gekennzeichnet, dass** der Wirkstoffkomplex zu 10 Gewichtsprozenten aus Glycin besteht und der Schmelzbereich des Wirkstoffkomplexes eine endothermische Peaktemperatur von 148 ± 2°C und eine exothermische Peaktemperatur von 153 ± 2°C aufweist.

6. Wirkstoffkomplex bestehend aus einem Salz der o-Acetylsalicylsäure mit einer basischen Aminosäure und Glycin, erhältlich, indem o-Acetylsalicylsäure und eine basische Aminosäure in Wasser oder einem mit Wasser mischbaren organischen Lösungsmittel bei einer Temperatur kleiner gleich 40°C rasch zusammengegeben werden, die homogene Mischung anschließend auf -5 bis 10°C abgekühlt wird, danach gekühltes Aceton und eine gekühlte Glycin-Suspension dazugegeben werden, mindestens 1 Stunde lang nachgerührt wird, das Kristallisat isoliert wird und während der Kristallisation eine Temperatur von 5°C nicht überschritten wird, und der Feststoff eine mittlere Korngröße unterhalb von 100 µm aufweist.

7. Wirkstoffkomplex gemäß Anspruch 6, **dadurch gekennzeichnet, dass** der Wirkstoffkomplex zu 8 bis 12 Gewichtsprozenten aus Glycin besteht.

8. Wirkstoffkomplex gemäß einem der Ansprüche 6 bis 7 mit Lysin als basische Aminosäure.

9. Wirkstoffkomplex gemäß einem der Ansprüche 6 bis 7 mit D,L-Lysin als basische Aminosäure.

10. Wirkstoffkomplex gemäß Anspruch 9, **dadurch gekennzeichnet, dass** der Wirkstoffkomplex zu 10 Gewichtsprozenten aus Glycin besteht und der Schmelzbereich eine endothermische Peaktemperatur von 148 ± 2°C und eine exothermische Peaktemperatur von 153 ± 2°C aufweist.

11. Wirkstoffkomplex gemäß einem der Ansprüche 6 bis 10, **dadurch gekennzeichnet, dass** während der Kristallisation die Rührenergie nicht mehr als 0,1 W pro Liter Reaktionsmedium beträgt.

12. Wirkstoffkomplex gemäß einem der Ansprüche 6 bis 11, **dadurch gekennzeichnet, dass** das Verfahren unter sterilen Bedingungen durchgeführt wird.

13. Verfahren zur Herstellung eines Stoffes wie in einem der Ansprüche 1 bis 12 genannt, **dadurch gekennzeichnet, dass** o-Acetylsalicylsäure und eine basische Aminosäure in Wasser oder einem mit Wasser mischbaren organischen Lösungsmittel bei einer Temperatur kleiner gleich 40°C rasch zusammengegeben werden, die homogene Mischung anschließend auf -5 bis 10°C abgekühlt wird, danach gekühltes Aceton und eine gekühlte Glycin-Suspension dazugegeben werden, mindestens 1 Stunde lang nachgerührt wird, das Kristallisat isoliert wird und während der Kristallisation eine Temperatur von 5°C nicht überschritten wird.

14. Verfahren nach Anspruch 13, **dadurch gekennzeichnet, dass** während der Kristallisation die Rührenergie nicht mehr als 0,1 W pro Liter Reaktionsmedium beträgt.

15. Verfahren nach einem der Ansprüche 13 bis 14, **dadurch gekennzeichnet, dass** das Verfahren unter sterilen Bedingungen durchgeführt wird.

16. Arzneimittel enthaltend mindestens einen Wirkstoffkomplex gemäß einem der Ansprüche 1 bis 12.

17. Arzneimittel gemäß Anspruch 16, **dadurch gekennzeichnet, dass** es sich um eine parenterale Applikationsform handelt.

18. Arzneimittel gemäß einem der Ansprüche 16 bis 17, **dadurch gekennzeichnet, dass** es sich um eine Injektions- und Infusionszubereitung in Form einer Lösung, Suspension oder Emulsion handelt.

19. Arzneimittel gemäß einem der Ansprüche 16 bis 18, **dadurch gekennzeichnet, dass** es sich um eine Applikationsform handelt, in der der Wirkstoffkomplex beispielsweise als Lyophilisat oder steriles Pulver getrennt von dem Injektions- oder Infusionslösungsmittel vorliegt und erst kurz vor der Applikation durch Mischen mit dem Lösungsmittel die fertige Injektions- oder Infusionszubereitung hergestellt werden kann.

20. Arzneimittel gemäß einem der Ansprüche 16 bis 19 zur Behandlung von Arthritis, Neuralgien, Myalgien, Migräne, myocardialem Infarkt, Schlaganfall, ischämischen Herzkrankheiten, Angina pectoris, Bypass-Operationen, PTCA Stent-Implantation und/oder von Asthma, wobei Asthma durch Inhalation des Wirkstoffkomplexes zu behandeln ist.

## Claims

1. Active compound complex consisting of a salt of o-acetylsalicylic acid with a basic amino acid and glycine, **characterized in that** the particle size distribution has a mean particle size of less than 100 µm.

2. Active compound complex according to Claim 1, **characterized in that** the active compound complex comprises 8 to 12 per cent by weight of glycine.

3. Active compound complex according to Claim 1 or 2 with lysine as basic amino acid.

4. Active compound complex according to any one of Claims 1 to 3 with D,L-lysine as basic amino acid.

5. Active compound complex according to Claim 4, **characterized in that** the active compound complex comprises 10 per cent by weight of glycine and the melting range of the active compound complex has an endothermal peak temperature of 148 ± 2°C and an exothermal peak temperature of 153 ± 2°C.

6. Active compound complex consisting of a salt of o-acetylsalicylic acid with a basic amino acid and glycine, obtainable by combining o-acetylsalicylic acid and a basic amino acid rapidly in water or a water-miscible organic solvent at a temperature of less than or equal to 40°C, then cooling the homogeneous mixture to from -5 to 10°C, then adding cooled acetone and a cooled glycine suspension, stirring for at least 1 more hour, isolating the crystals and, during crystallization, not exceeding a temperature of 5°C, the active compound complex having a mean particle size of less than 100 µm.

7. Active compound complex according to Claim 6, **characterized in that** the active compound complex comprises 8 to 12% by weight of glycine.

8. Active compound complex according to Claim 6 or 7 with lysine as basic amino acid.

9. Active compound complex according to Claim 6 or 7 with D,L-lysine as basic amino acid.

10. Active compound complex according to Claim 9, **characterized in that** the active compound complex comprises 10% by weight of glycine and the melting range has an endothermal peak temperature of 148 ± 2°C and a exothermal peak temperature of 153 ± 2°C.

11. Active compound complex according to any of Claims 6 to 10, **characterized in that** during crystallization the stirring energy is not more than 0.1 W per litre of reaction medium.

12. Active compound complex according to any of Claims 6 to 11, **characterized in that** the process is carried out under sterile conditions.

13. Process for preparing a substance as mentioned in any of Claims 1 to 12, **characterized in that** o-acetylsalicylic acid and a basic amino acid are combined rapidly in water or a water-miscible organic solvent at a temperature of less than or equal to 40°C, the homogeneous mixture is then cooled to from -5 to 10°C, cooled acetone and a cooled glycine suspension are then added, stirring is continued for at least 1 hour, the crystals are isolated and, during crystallization, a temperature of 5°C is not exceeded.

14. Process according to Claim 13, **characterized in that** during crystallization the stirring energy is not more than 0.1 W per litre of reaction medium.

15. Process according to Claim 13 or 14, **characterized in that** the process is carried out under sterile conditions.

16. Medicament comprising at least one active compound complex according to any of Claims 1 to 12.

17. Medicament according to Claim 16, **characterized in that** it is a parenteral administration form.

18. Medicament according to Claim 16 or 17, **characterized in that** it is a preparation for injection and infusion in the form of a solution, suspension or emulsion.

19. Medicament according to any of Claims 16 to 18, **characterized in that** it is an application form in which the active compound complex is present, for example, as a lyophilisate or sterile powder, separately from the solvent for injection or infusion, and the finished preparation for injection or infusion is prepared only shortly before the administration by mixing with the solvent.

20. Medicament according to any of Claims 16 to 19 for the treatment of arthritis, neuralgia, myalgia, migraine, myocardial infarction, stroke, ischemic heart diseases, angina pectoris, bypass operations, PTCA stent implantation and/or of asthma, where asthma is to be treated by inhalation of the active compound complex.

## Revendications

1. Complexe de principes actifs constitué d'un sel de l'acide o-acétylsalicylique, avec un acide aminé basique et de la glycine, **caractérisé en ce que** la distribution granulométrique présente une granulométrie moyenne inférieure à 100 µm.

2. Complexe de principes actifs selon la revendication 1, **caractérisé en ce que** le complexe de principes actifs est pour 8 à 12 % en poids constitué de glycine.

3. Complexe de principes actifs selon l'une des revendications 1 à 2, contenant de la lysine en tant qu'acide aminé basique.

4. Complexe de principes actifs selon l'une des revendications 1 à 3, contenant de la D,L-lysine en tant qu'acide aminé basique.

5. Complexe de principes actifs selon la revendication 4, **caractérisé en ce que** le complexe de principes actifs est pour 10 % en poids constitué de glycine, la plage de fusion du complexe de principes actifs présentant une température endothermique maximale de 148 ± 2°C et une température exothermique maximale de 153 ± 2°C.

6. Complexe de principes actifs, constitué d'un sel de l'acide o-acétylsalicylique, avec un acide aminé basique et de la glycine, pouvant être obtenu par réunion rapide, à une température inférieure ou égale à 40°C, d'acide o-acétylsalicylique et d'un acide aminé basique dans de l'eau ou dans un solvant organique miscible à l'eau, puis refroidissement du mélange homogène à -5 à 10°C, puis addition d'acétone refroidie et d'une suspension refroidie de glycine, agitation pendant encore au moins 1 heure, isolement du cristallisat, sans dépasser pendant la cristallisation une température de 5°C, le complexe de principes actifs présentant une granulométrie moyenne inférieure à 100 µm.

7. Complexe de principes actifs selon la revendication 6, **caractérisé en ce que** le complexe de principes actifs est pour 8 à 12 % en poids constitué de glycine.

8. Complexe de principes actifs selon l'une des revendications 6 à 7, contenant de la lysine en tant qu'acide aminé basique.

9. Complexe de principes actifs selon l'une des revendications 6 à 7, contenant de la D,L-lysine en tant qu'acide aminé basique.

10. Complexe de principes actifs selon la revendication 9, **caractérisé en ce que** le complexe de principes actifs est pour 10 % en poids constitué de glycine, la plage de fusion présentant une température endothermique maximale de 148 ± 2°C et une température exothermique maximale de 153 ± 2°C.

11. Complexe de principes actifs selon l'une des revendications 6 à 10, **caractérisé en ce que**, pendant la cristallisation, l'énergie d'agitation n'est pas supérieure à 0,1 W par litre de milieu réactionnel.

12. Complexe de principes actifs selon l'une des revendications 6 à 11, **caractérisé en ce que** le procédé est mis en oeuvre dans des conditions stériles.

13. Procédé de préparation d'une matière telle que mentionnée dans l'une des revendications 1 à 12, **caractérisé en ce qu'**on réunit rapidement à une température inférieure à 40°C de l'acide o-acétylsalicylique et un acide aminé basique dans de l'eau ou dans un solvant organique miscible à l'eau, puis on refroidit le mélange homogène à -5 à 10°C, puis on ajoute de l'acétone refroidie et une suspension refroidie de glycine, on agite encore pendant au moins 1 heure, on isole le cristallisat et, pendant la cristallisation, on ne dépasse pas une température de 5°C.

14. Procédé selon la revendication 13, **caractérisé en ce que**, pendant la cristallisation, l'énergie d'agitation n'est pas supérieure à 0,1 W par litre de milieu réactionnel.

15. Procédé selon l'une des revendications 13 à 14, **caractérisé en ce que** le procédé est mis en oeuvre dans des conditions stériles.

16. Médicament contenant au moins un complexe de principes actifs selon l'une des revendications 1 à 12.

17. Médicament selon la revendication 16, **caractérisé en ce qu'**il s'agit d'une forme galénique parentérale.

18. Médicament selon l'une des revendications 16 à 17, **caractérisé en ce qu'**il s'agit d'une préparation injectable et pour perfusion, sous forme d'une solution, d'une suspension ou d'une émulsion.

19. Médicament selon l'une des revendications 16 à 18, **caractérisé en ce qu'**il s'agit d'une forme galénique dans laquelle le complexe de principes actifs se présente par exemple sous forme d'un lyophilisat ou d'une poudre stérile, séparée du solvant pour injection ou pour perfusion, et que la préparation injectable ou pour infusion finie ne peut être préparée que peu de temps avant administration, par mélange avec le solvant.

20. Médicament selon l'une des revendications 16 à 19, pour le traitement de l'arthrite, des névralgies, des myalgies, des migraines, de l'infarctus du myocarde, de l'accident vasculaire cérébral, de la cardiopathie ischémique, de l'angine de poitrine, des opérations de pontage, de l'implantation d'un stent par ACTP et/ou de l'asthme, l'asthme devant être traité par inhalation du complexe de principes actifs.
